(19) Europäisches Patentamt
European Patent Office
Office européen des brevets

(11) Publication number: **0 228 564 B1**

(12) # EUROPEAN PATENT SPECIFICATION

(45) Date of publication of patent specification: **29.01.92**

(51) Int. Cl.5: **C07C 241/00**, C07C 315/00, C07C 317/00, C07C 323/00, C07D 317/68, A01N 37/28, A01N 37/40, A01N 43/30

(21) Application number: **86116280.8**

(22) Date of filing: **24.11.86**

(54) **Novel insecticidal diacylhydrazine compounds.**

(30) Priority: **09.12.85 US 806995**

(43) Date of publication of application:
**15.07.87 Bulletin 87/29**

(45) Publication of the grant of the patent:
**29.01.92 Bulletin 92/05**

(84) Designated Contracting States:
**AT BE CH DE ES FR GB GR IT LI LU NL SE**

(56) References cited:
**EP-A- 0 035 619**
**EP-A- 0 232 975**
**FR-M- 8 005**
**US-A- 2 758 054**

**CHEMICAL ABSTRACTS, vol. 95, no. 15, October 12, 1981, page 606, ref. no. 132148a, Columbus, Ohio, US; I. D. KALIKHMAN et al, "Z,E-isomerism of aromatic acid alpha-methyl-hydrazides"**

(73) Proprietor: **AMERICAN CYANAMID COMPANY**
**1937 West Main Street P.O. Box 60**
**Stamford Connecticut 06904-0060(US)**

(72) Inventor: **Addor, Roger Williams**
**P.O. Box 347 A RR 1**
**Pennington New Jersey 08534(US)**
Inventor: **Kuhn, David George**
**Adrian Place**
**Newtown Pennsylvania 18940(US)**
Inventor: **Wright, Donald Perry, Jr.**
**RR 1, Box 294 Bl.Woosamonsa Road**
**Pennington New Jersey 08534(US)**

(74) Representative: **Wächtershäuser, Günter, Dr.**
**Tal 29**
**W-8000 München 2(DE)**

EP 0 228 564 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 82, no. 7, February 17, 1975, page 127, ref. no. 39589s, Columbus, Ohio, US; N. ONODERA et al, "Benzoylhydrazine derivatives as acaricides" & JP-A-74 47528

JOURNAL OF PHARMACEUTICAL SCIENCES, vol. 57, no. 11, November 1968, pp. 2011-2012; M. R. WILEY et al, "Inhibition of liver-derived monoamine oxidase by several isopropyl hydrazines"

IL FARMACO, vol. 31, no. 7, July 1976, pp. 517-526; G. MAZZONE et al, "Ricerche nel campo degli inibitori della monoaminossidasi: Sintesi de etilsiringoilidrazidi - N-alchil E -N-aralchilsostituite"

## Description

The present invention provides novel compounds having the structural formula (I)

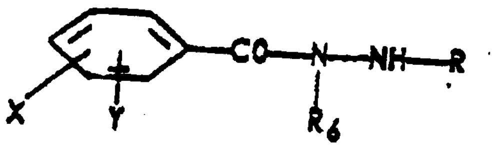

(I)

wherein R is hydrogen, $C_2$-$C_6$ alkyl or $C_5$-$C_6$ cycloalkyl, $R_6$ is tert-butyl; X and Y are each independently H, $C_1$-$C_3$ alkyl , $C_1$-$C_3$ alkoxy, $C_1$-$C_3$ alkylthio, $C_1$-$C_3$ alkyl-sulfinyl, $C_1$-$C_3$ alkylsulfonyl, cyano, F, Cl, Br, nitro, $CF_3$, $R_1CF_2Z$-, 1,1-difluoro-2,2-dichloroethoxy, $R_2CO$ or $R_3R_4N$; Z is $S(O)_n$ or O; $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl; $R_4$ is H, $C_1$-$C_3$ alkyl or $R_5CO$; $R_5$ is H or $C_1$-$C_3$ alkyl and n is 0, 1 or 2; with the provisos that when R is tert-butyl and Y is chloro in the para position of the ring, X is a substituent other than hydrogen, and when X and Y are both H, R is not hydrogen.

The present invention also relates to an insecticidal composition comprising an insecticidally effective amount of a compound of the formula (II)

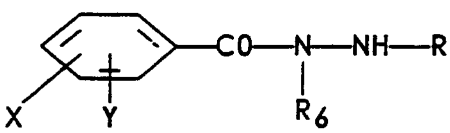

(II)

wherein R and $R_6$ are each independently hydrogen, $C_2$-$C_6$ alkyl or $C_5$-$C_6$ cycloalkyl; X and Y are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, I, nitro, $CF_3$, $R_1CF_2Z$-, 1,1-difluoro-2,2-dichloroethoxy, $R_2CO$ or $R_3R_4N$ and when taken together X and Y may form a ring in which XY are represented by the structure:

$-OCH_2O-$, $-OCF_2O-$ or 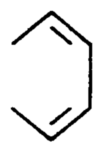 ;

Z is S(O)n or O; $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl; $R_4$ is H, $C_1$-$C_3$ alkyl or $R_5CO$; $R_5$ is H or $C_1$-$C_3$ alkyl and n is 0, 1 or 2; with the provisos that when R is hydrogen, $R_6$ is $C_2$-$C_5$ alkyl or $C_5$-$C_6$ cycloalkyl and when $R_6$ is hydrogen, R is $C_2$-$C_5$ alkyl or $C_5$-$C_6$ cycloalkyl; and provided also that when R is tert-butyl and Y is chloro in the para position of the ring, X is a substituent other then hydrogen.

The substituted formula (II) benzoic acid 1-alkyl-, 2-alkyl- and 2-cycloalkylhydrazides, like the formula (I), 1-alkyl-, 2-alkyl-, and 2-cycloalkylhydrazides, are potent stomach poisons. As such, these formula (I) and formula (II) hydrazides effectively control insect populations and protect plants from insect attack. Insecticidally-effective amounts of the active compound can be applied to the foliage of plants upon which the insects feed or to the soil, water or other media in which said plants are growing. These compounds may also be made available to the insects in the form of baits or applied to the insects' breeding grounds and habitat.

Additionally, many of the formula (II) benzoic acid 1-alkyl-, 2-alkyl- and 2-cycloalkylhydrazides, substituted with halogen, $CH_3$, $CF_3$, $-OCH_3$, $-OCH_2O-$, $OCF_2O-$, $NH_2$, $NO_2$ or -CH=CH-CH=CH-, are useful as intermediates for the preparation of dibenzoylhydrazines which are effective as insecticidal agents and systemic soil insecticidal agents.

Process of Manufacture

The formula (I) 2-alkyl- and 2-cycloalkylhydrazides of the invention are prepared by reacting an alkyl- or cycloalkylhydrazine hydrohalide with a benzoylhalide in the presence of aqueous base.

Generally, the alkyl or cycloalkylhydrazine hydrohalide is dispersed in an organic solvent, such as methylene chloride, ether or the like, and the resulting mixture then admixed with aqueous base. Usually, about two to six molar equivalents of base, such as sodium carbonate or sodium hydroxide, per equivalent of alkylhydrazine hydrohalide are used to achieve the benzoylation of the alkyl hydrazine. The thus-prepared mixture is then admixed with the appropriate benzoyl halide dissolved or dispersed in an organic solvent, preferably the same solvent used for dissolution of the alkyl- or cycloalkyl- hydrazine hydrohalide.

The mixture is stirred or agitated for a sufficient period of time to form the benzoic acid alkyl-cycloalkylhydrazide which is readily recovered from the mixture by separation of the aqueous phase from the organic phase and evaporation of the organic solvent from said organic phase.

The reaction is graphically illustrated below:

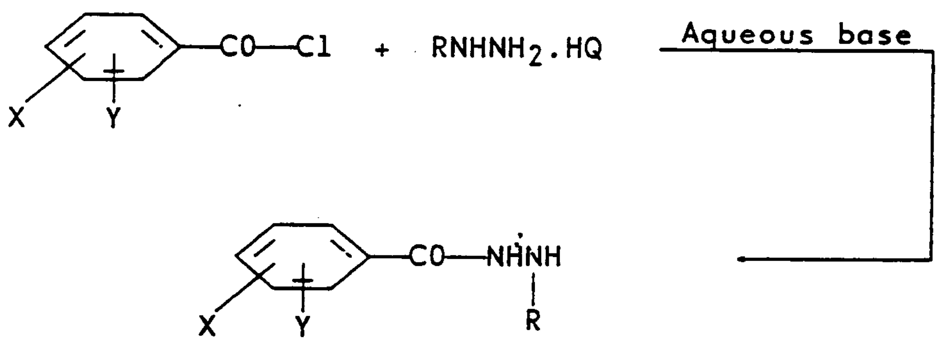


wherein Q is halogen, preferably chlorine and R, X and Y are as described for formula (I) compounds illustrated hereinabove.

The formula (II) benzoic acid, 1-alkyl and 2-cycloalkylhydrazides of this invention, depicted by the structure:

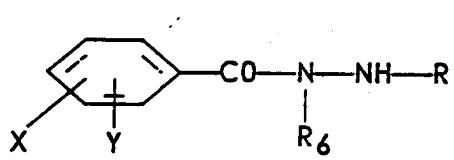

(II)

wherein R is hydrogen; $R_6$ is $C_2$-$C_6$ alkyl or $C_5$-$C_6$ cycloalkyl and X and Y are as described hereinabove, are prepared by reacting an alkylhydrazine or cycloalkylhydrazine with acetone. After the mixture is permitted to stand for a short period of time, it is treated with ether and potassium hydroxide pellets. The ether layer is separated from the mixture and evaporated to yield the 1-alkyl-2-isopropylidenehydrazide or 1-cycloalkyl-2-isopropylidenehydrazide.

The resulting 1-alkyl- or 1-cycloalkyl-2-isopropylidene hydrazide then is reacted with a benzoyl halide in the presence of 10% sodium hydroxide to yield 1-alkyl or 1-cycloalkyl 2-isopropylidenehydrazide of benzoic acid. Hydrolysis of the thus-formed product with a dilute mineral acid such as 10% HCl in the presence of alcohol yields the 1-alkyl or 1-cycloalkylhydrazide of benzoic acid.

These reactions are illustrated as follows:

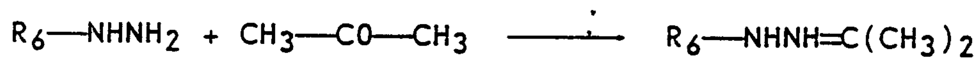

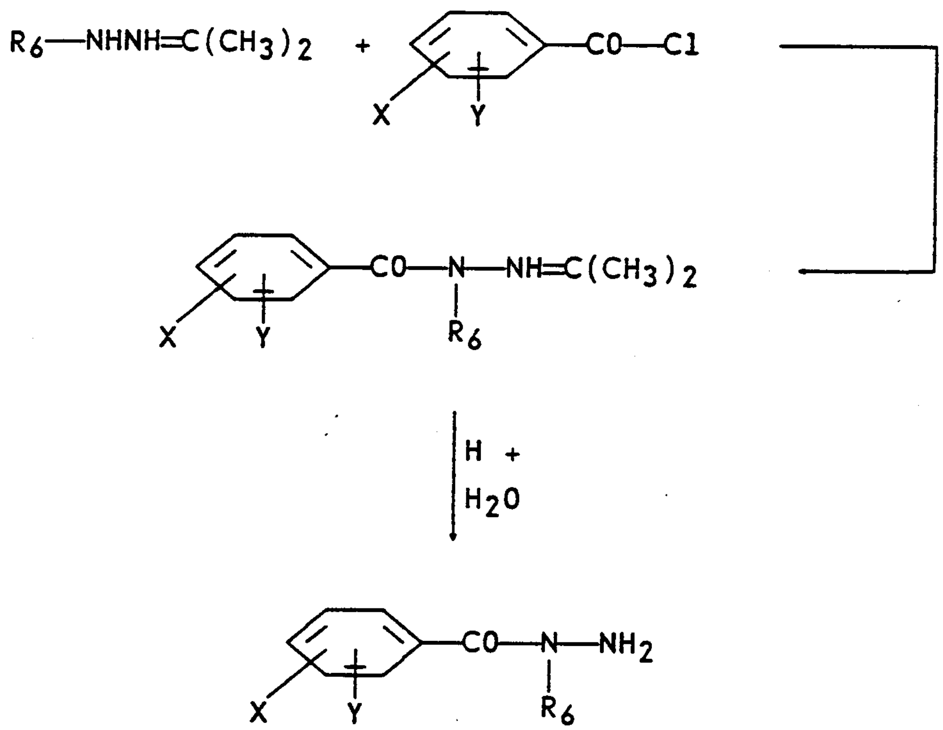


wherein $R_6$, X and Y are as described hereinabove.

Preparation of the formula (II) benzoic acid alkyl-and cycloalkylhydrazides of this invention, wherein $R_6$ is hydrogen, also is achieved by reduction of the appropriate benzoic acid alkylidene hydrazide with hydrogen in the presence of a noble metal catalyst, such as platinum or palladium. The reaction preferably is conducted in the presence of a lower alkyl ($C_1$-$C_4$) alcohol under a blanket of hydrogen maintained at about 20 to 60 psig. The reaction is illustrated below:

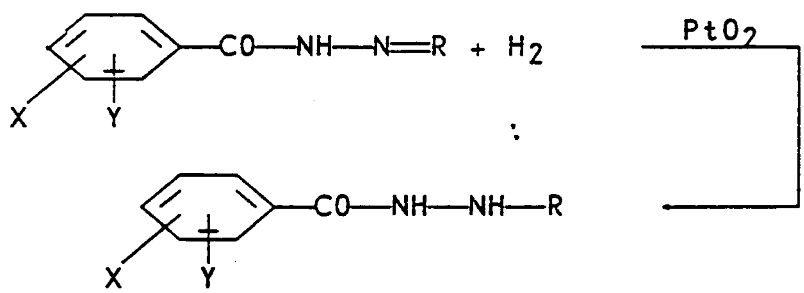


(II)

wherein $R_1$, X and Y are as described for formula (II) compounds hereinabove.

Formula (II) benzoic acid 2-alkylhydrazides and 2-cycloalkylhydrazides are useful as intermediates for the preparation of dibenzoylhydrazine compounds illustrated by formula given below, which are found to be extremely potent insect stomach poisons and systemic insecticidal agents. These dibenzoylhydrazines are effective for controlling a variety of insects including, but not limited to Lepidoptera, Homoptera, Orthoptera, Coleoptera and Diptera, and are likewise effective for protecting a variety of crops from insect attack.

The said dibenzoylhydrazine compounds have the following structure:

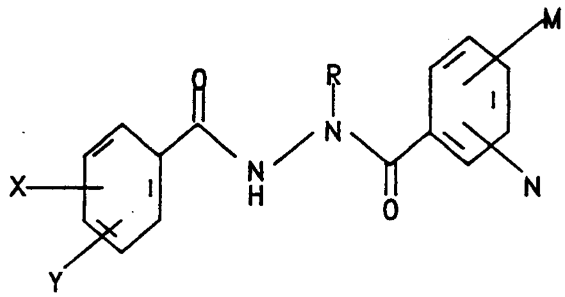

wherein R is $C_2$-$C_6$ alkyl or $C_5$-$C_6$ cycloalkyl; X, Y, M and N are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, I, nitro, $CF_3$ or $R_1CF_2Z$-, 1,1-difluoro-2,2-dichloroethoxy, $R_2CO$ or $R_3R_4N$, and when taken together, X and Y may form a ring in which XY are represented by the structure:

$-OCH_2O-$, $-OCF_2O-$ or 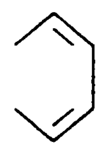 ;

and when taken together, M and N may form a ring in which MN are represented by the structure:

$-OCH_2O-$, $-OCF_2O-$ or 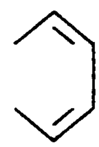 ;

Z is S(O)n or O; $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl; $R_4$ is H, $C_1$-$C_3$ alkyl or $R_5CO$; $R_5$ is H or $C_1$-$C_3$ alkyl and n is 0, 1 or 2; with the provisos that at least one of X, Y, M or N is a substituent other than hydrogen and when M is para nitro, at least one other of X, Y, or N must be a substituent other then hydrogen.

These compounds are prepared by the reacting approximately equimolar amounts of a benzoic acid alkylhydrazide and a benzoyl halide in the presence of an aprotic solvent, such as an ether, chlorinated hydrocarbon or the like and aqueous base. Generally, about two to six molar equivalents of base per equivalent of benzoic acid alkylhydrazide are sufficient to bring the reaction to completion. The reaction is graphically illustrated below:

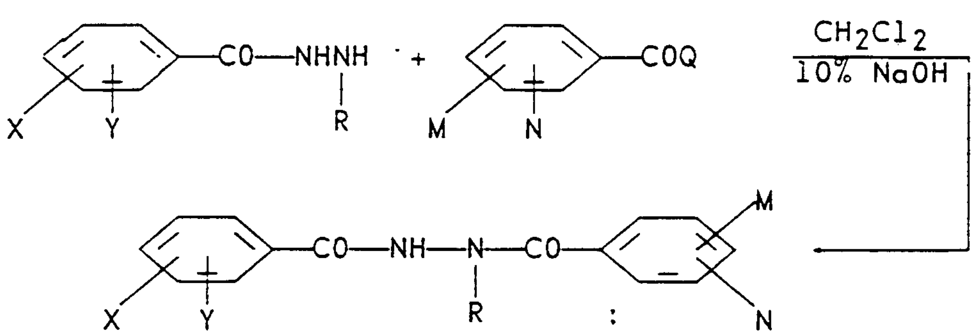


wherein Q is halogen; and R, X, Y, M and N are as described hereinabove.

As stated hereinabove, advantageously, the compounds of this invention can be prepared in a rather expeditious manner. In accordance with the process of this invention, a $C_2$-$C_6$ alkylhydrazine hydrohalide is

dispersed in an organic solvent, such as methylene chloride, ether or the like, and the resulting mixture then admixed with an aqueous base. Usually, about two to six molar equivalents of base, such as sodium carbonate or sodium hydroxide, per equivalent of alkylhydrazine hydrohalide are used to achieve the benzoylation of the alkyl hydrazine. The thus-prepared mixture is then admixed with the appropriate benzoyl halide dissolved or dispersed in an organic solvent, preferably the same solvent used for dissolution of the alkylhydrazine hydrohalide.

The mixture is stirred or agitated for a sufficient period of time to form the benzoic acid alkylhydrazide which is readily recovered from the mixture by separation of the aqueous phase from the organic phase and evaporation of the organic solvent from said organic phase.

The reaction is graphically illustrated below:

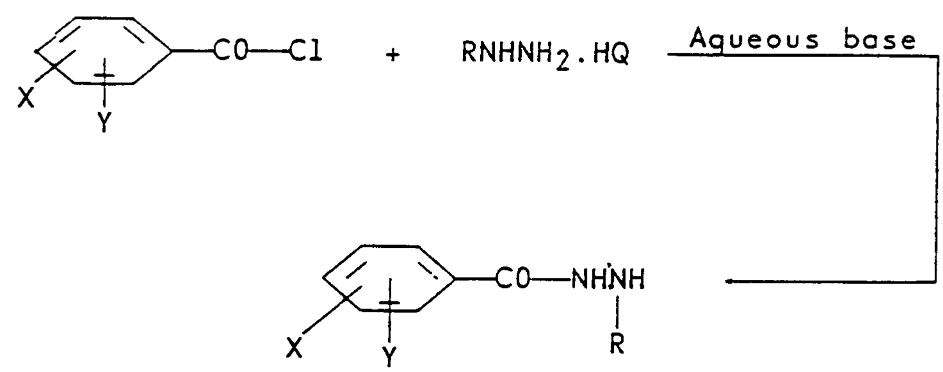


wherein Q is halogen, preferably chlorine; R is $C_2$-$C_6$ alkyl and X and Y are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, nitro, $CF_3$, $R_1CF_2Z$-, 1,1-difluoro-2,2-dichloroethoxy, $R_2CO$ or $R_3R_4N$ and when taken together X and Y may form a ring in which XY are represented by the structure:

$-OCH_2O-$, $-OCF_2O-$ or 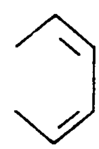 ;

Z is S(O)n or O and $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl and $R_4$ is H, $C_1$-$C_3$ alkyl or $R_5CO$; $R_5$ is H or $C_1$-$C_3$ alkyl and n is 0, 1 or 2. As used in this application, where R is $C_2$-$C_6$ alkyl, it is intended to include straight and branched alkyl groups $C_2$-$C_6$ and cycloalkyl groups $C_3$-$C_6$.

Preparation of the benzoic acid alkylhydrazides may also be achieved by reduction of the appropriate benzoic acid alkylidene hydrazide with hydrogen in the presence of a noble metal catalyst, such as platinum or palladium. The reaction is preferably conducted in the presence of a lower alkyl $C_1$-$C_4$ alcohol under a blanket of hydrogen maintained at about 20 to 60 psig. The reaction is illustrated below:

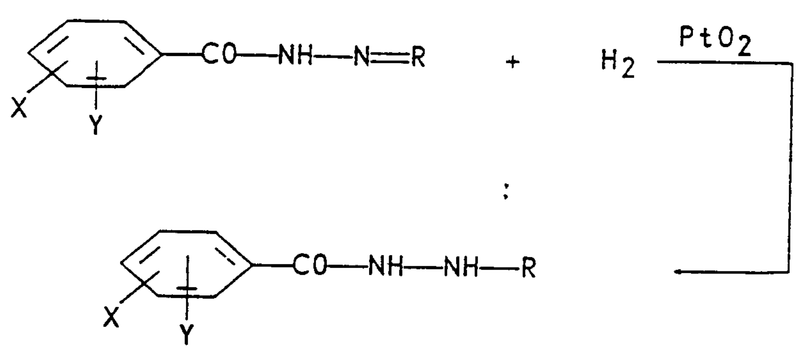

wherein R is $C_2$-$C_6$ alkyl; X and Y are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, nitro, $CF_3$, $R_1CF_2Z$-, 1,1-difluoro-2,2-dichloroethoxy, $R_2CO$ or $R_3R_4N$, and when taken together X and Y may form a ring in which XY are represented by the structure:

$-OCH_2O-$, $-OCF_2O-$ or 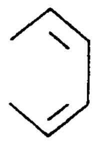 ;

Z is S(O) n or O and $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl and $R_4$ is H, $C_1$-$C_3$ alkyl or $R_5CO$; $R_5$ is H or $C_1$-$C_3$ alkyl and n is 0, 1 or 2.

The above-described benzoic acid alkylhydrazides are useful as insecticidal agents and particularly effective when used for the control of lepidopterous insects. These compounds also have the added advantage that they are useful as intermediates for the preparation of dibenzoyl hydrazine compounds which we have found to be extremely potent insect stomach poisons and systemic insecticidal agents, effective for controlling a variety of insects including, Lepidoptera, Homoptera, Orthoptera, Coleoptera and Diptera, and are likewise effective for protecting a variety of crops from insect attack. These compounds have also been found to have some activity as contact insecticides.

## Formulations

As previously indicated, benzoic acid 1-alkyl, 2-alkyl and 2-cycloalkylhydrazides of this invention are excellent insect stomach poisons and have contact insecticidal activity. They are effective when applied to the foliage of plants which are to be protected from insect attack or when applied to the breeding grounds, food supply or habitat of insects.

Emulsifiable concentrates, wet and dry flowable compositions, granular formulations, microemulsions and wettable powders all lend themselves to soil and/or water application and provide the desired insect control and requisite plant protection.

In practice, protection of living plants is achieved by applying to the foliage of said plants and/or to the soil, water or other media in which they are growing, about 0.1 kg/hectare to about 10.0 kg/hectare, preferably about 0.28 to 4.0 kg/hectare, of the formula (I) or formula (II) benzoic acid 2-alkyl- or 2-cycloalkylhydrazide. Advantageously, application of these formulations to the insects habitat, food supply and/or breeding grounds at the above said rates also provide control of insect populations in the treated area.

If the active formula (I) or formula (II) compound is applied as a dilute spray, said spray should contain 10 ppm to about 10,000 ppm of the active ingredient to provide the desired protection and insect control.

A typical emulsifiable concentrate formulation is prepared by dispersing about 30% w/v of the benzoic acid 1-alkyl-, 2-alkyl and 2-cycloalkylhydrazide of the invention in 50% w/v of 2-pyrrolidone; in 10% w/v of n-butanol; 7% w/v of a polyalkylene glycol ether, such as POLYFAR® S320 manufactured by Westvaco-Polychemicals, Charleston Heights, South Carolina; and 3.0% w/v of nonylphenoxy polyethoxy ethanol offered by Rohm and Haas Co as TRITON® N101.

Emulsifiable concentrates are especially useful for distributing the active benzoic acid 1-alkyl, 2-alkyl and 2-cycloalkylhydrazides of this invention since they are readily dispersed in water for application as liquid sprays. Such emulsifiable concentrates also may be added to irrigation water or flooded paddies, such as used for rice cultivation, or they may be applied directly to the plants or the locus to be protected from insect infestation using aerial applicators or ground equipment designed for ultra low volume (ULV) or low volume (LV) application of the undiluted concentrates as finely divided discrete droplets.

Granular formulations may be prepared by dissolving the active formula (I) or formula (II) hydrazide in a lowboiling solvent, such as methylene chloride, and spraying the thus-prepared solution on a sorptive carrier such as kaolin, bentonite, attapulgite, montmorillonite or the like, in sufficient amount to provide from about 2% to 20%, preferably about 3% to 15% by weight, of active ingredient based on the total weight of the granulated product.

Wettable powder formulations can be prepared by grinding together about 30% to 75% by weight of the active formula (I) or (II) hydrazide with about 5% to 10% by weight of an anionic surfactant, such as a naphthalene sulfonate condensate or a sodium or ammonium salt of a condensed mono naphthalene

sulfonic acid; about 3% to 5% by weight of an anionic surfactant such as an alkyl naphthalene sulfonate, i.e. sodium di-n-butyl naphthalene sulfonate, sodium diisopropyl naphthalene sulfonate or the like; and the remainder of the composition an inert diluent such as attapulgite, kaolin, montmorillonite, talc, diatomaceous earth or the like.

The following examples are presented herein simply as illustrations of the present invention and are not limitative thereof.

EXAMPLE 1

Preparation of benzoic acid, 2-tert-butylhydrazide

tert-Butylhydrazine hydrochloride (15.6 g, 0.125 mole) is dissolved in 350 mL of methylene chloride. To this solution is added 240 mL of 10% aqueous sodium hydroxide (24 g, 0.60 mole). A solution of benzoyl chloride (d = 1.211, 14.5 mL, 17.6 g, 0.125 mole) in methylene chloride is then added at moderate rate to the rapidly stirring two-phase system.

After stirring the mixture for 24 hours at ambient temperatures, the methylene chloride phase is removed, washed with 5% aqueous sodium hydroxide, water, saturated sodium chloride solution, and then dried over sodium sulfate. Evaporation in vacuo gives 19.3 g of white solid, mp 87-94°C, which is recrystallized from 2-propanol-water to give 13.0 g of product, mp 92-94°C.

Substituting p-chlorobenzoyl chloride for benzoyl chloride in the above reaction yields p-chlorobenzoic acid, 2-tert-butylhydrazide; melting point 116-122°C. Similarly, substituting p-fluorobenzoyl chloride, p-nitrobenzoyl chloride, o-toluyl chloride, m-fluorobenzoyl chloride, p-bromobenzoyl chloride, p-trifluoromethylbenzoyl chloride, o-anisyl chloride, p-toluyl chloride, o-chlorobenzoyl chloride, p-iodobenzoyl chloride, o-iodobenzoyl chloride, p-ethylbenzoyl chloride and o-fluorobenzoyl chloride, o-nitrobenzoyl chloride, for benzoyl chloride, yields respectively: p-fluorobenzoic acid, o-bromobenzoyl chloride, 2-tert-butylhydrazide; mp 136-138°C; p-nitrobenzoic acid, 2-tert-butylhydrazide; o-toluic acid, 2-tert-butylhydrazide; m-fluorobenzoic acid, 2-tert-butylhydrazide, mp 119-120°C; p-bromobenzoic acid, 2-tert-butylhydrazide; p-trifluoromethylbenzoic acid, 2-tert-butylhydrazide; anisic acid, 2-tert-butylhydrazide; p-toluic acid, 2-tert-butylhydrazide; o-chlorobenzoic acid, 2-tert-butylhydrazide; mp 68-70°C; o-iodobenzoic acid, 2-tert-butylhydrazide; p-ethylbenzoic acid, 2-tert-butylhydrazide; and o-fluorobenzoic acid, 2-tert-butylhydrazide; mp 58-59°C; o-nitrobenzoic acid, 2-tert-butylhydrazide; mp 116-118°C; o-bromobenzoic acid, 2-tert-butylhydrazide; mp 85-87°C; and N-methylanthranilic acid, 2-tert-butylhydrazide, mp 125-129°C.

The above reactions are illustrated below:

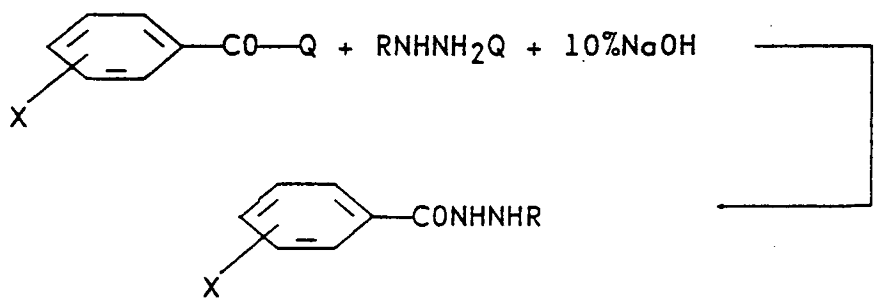


wherein Q is halogen, preferably chlorine; R is tert-butyl or tert-amyl. Other compounds that can be prepared by the above procedure using the appropriately substituted benzoyl halide include: m-chlorobenzoic acid, 2-tert-butylhydrazide, mp 120-123°C; p-cyanobenzoic acid, 2-tert-butylhydrazide, mp 135-136°C; anthranilic acid, 2-tert-butylhydrazide, mp 165-167°C; tert-butyl or isopropyl; and X is hydrogen, halogen, $C_1$-$C_3$ alkyl, methoxy, methylamino, $NH_2$, nitro or $CF_3$.

EXAMPLE 2

Preparation of 3,4-dichlorobenzoic acid, 2-tert-butylhydrazide

Tert-butylhydrazine hydrochloride (12.4 g 0.1 mole) is added to a solution of sodium carbonate (23.3 g, 0.22 mole) in 100 mL of water and 250 mL of ether. A solution of 3,4-dichlorobenzoyl chloride (20.9 g, 0.1 mole) in 50 mL of ether is then added dropwise at 0-15°C. After one hour, the reaction mixture is filtered

and the filtrate is separated. The organic layer is washed with 100 mL of water, dried over anhydrous $MgSO_4$, filtered and evaporated. Recrystallization of the residue from 2-propanol gives white crystals: yield 6.4 g, mp 144-145° C.

Following the above procedure, but substituting 2,4-dichlorobenzoyl chloride or 2,6-dichlorobenzoyl chloride for 3,4-dichlorobenzoyl chloride, yields, respectively 2,4-dichlorobenzoic acid, 2-tert-butylhydrazide, mp 115-117° C and 2,6-dichlorobenzoic acid, 2-tert-butylhydrazide, mp 173-174° C.

Similarly, substituting 2-chloro-4-nitrobenzoyl chloride, 3-bromo-4-methylbenzoyl chloride, 2,6-difluorobenzoyl chloride, 2,5-dichlorobenzoyl chloride, 3,5-dichlorobenzoyl chloride or naphthoyl chloride, for 3,4-dichlorobenzoyl chloride, yields respectively: 2-chloro-4-nitrobenzoic acid, 2-(tert)-butylhydrazide; 3-bromo-4-methylbenzoic acid, 2-(tert)-butylhydrazide; mp 95-97° C; 2,5-dichlorobenzoic acid, 2-(tert)-butylhydrazide and 3,5-dichlorobenzoic acid, 2-(tert)-butylhydrazide, mp 163-165° C; 1-naphthoic acid, 2-(tert)-butylhydrazide mp 148-150° C.

These reactions are illustrated below:

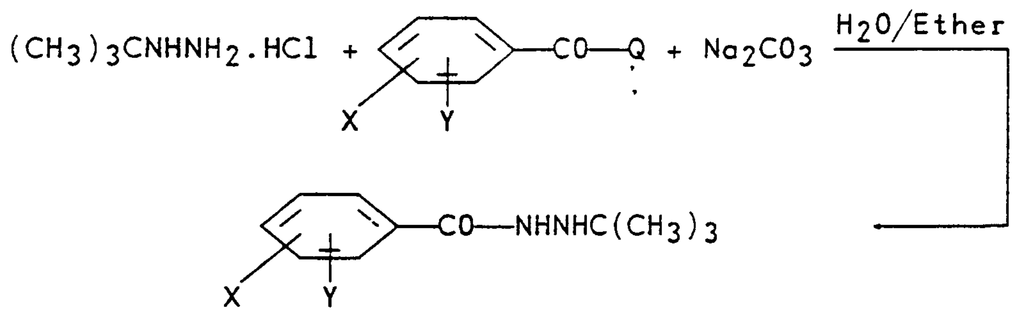


wherein Q is halogen, preferably chlorine; X and Y are each independently halogen, $C_1$-$C_3$ alkyl, methoxy, nitro or $CF_3$ and when taken together XY may represent

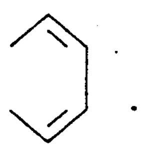

EXAMPLE 3

Preparation of benzoic acid, 3,4-dichloroisopropylidenehydrazine

3,4-Dichlorobenzoic acid hydrazide (11.7 g, 0.060 mole) is placed in the thimble of a Soxhlet extractor and flooded with hot acetone from an attached distillation flask. After overnight reflux, the acetone mixture concentrated under vacuum to afford a white solid. Recrystallization from ethyl acetate petroleum ether gives 9.5 g of the title compound as white crystals, mp 141-144° C.

This reaction is illustrated as below:

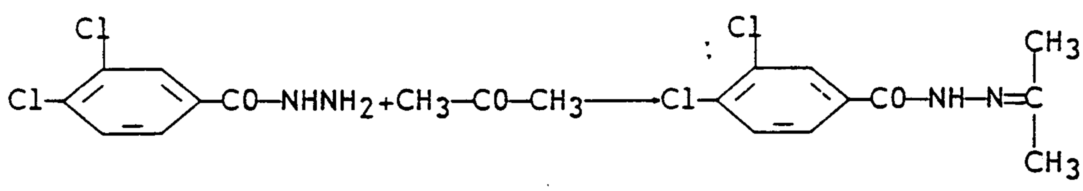

Following the above procedure but substituting the appropriate aldehyde or ketone for acetone yields the following compounds: benzoic acid (1-ethylpropylidene)hydrazide, mp 89-91° C; and benzoic acid (2,2-dimethylpropylidene)hydrazide, mp 168-169° C.

EXAMPLE 4

Preparation of 3,4-dichlorobenzoic acid, 2-isopropylhydrazide

3,4-Dichlorobenzoic acid isopropylidenehydrazide (9.2 g, 0.040 mole) and 100 mg of platinum oxide in 100 mL of methanol in a Parr hydrogenation apparatus is shaken for one hour and 30 minutes under an initial hydrogen pressure of 40 psig. The filtered reaction mixture is concentrated under vacuum and the resulting solids are recrystallized three times from isopropyl alcohol to give 2.6 g of the title compound as a white crystalline product, mp 112.5-115° C.

The reaction is illustrated below:

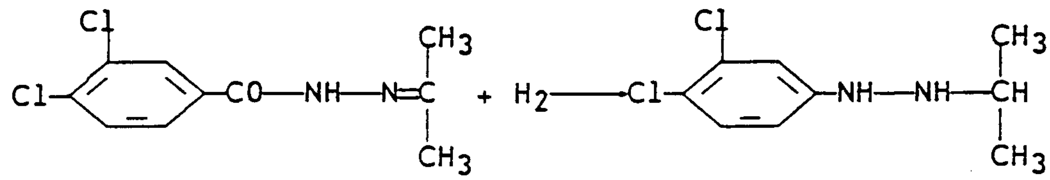

EXAMPLE 5

Preparation of acetone tert-butylhydrazone

To 6.6 g of acetone, cooled in an ice bath, is added 5.0 g of tert-butylhydrazine. The mixture is stirred and then allowed to stand for several minutes. Ether and potassium hydroxide pellets are then added to the mixture. The mixture is stirred, and then the ethereal layer is separated from the mixture. Distillation of the ethereal layer yields the product acetone tert-butylhydrazone b.p. 132-134° C.

The reaction is illustrated as follows:

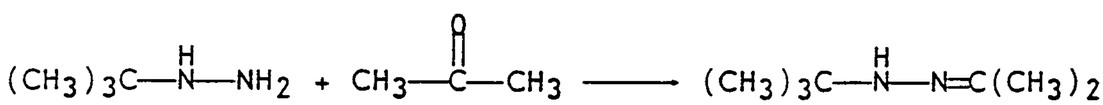

EXAMPLE 6

Preparation of 1-tert-butyl-2-isopropylidenehydrazide of benzoic acid

Acetone tert-butylhydrazone (2.0 g) is admixed with 4.4 g of benzoyl chloride and 15 mL of 10% sodium hydroxide. The mixture is stirred until the benzoyl chloride odor is no longer detectable. The resulting product is then dissolved in ether and dried over magnesium sulfate. Evaporation of the solvent from the mixture leaves acetone N-tert-butyl-N-benzoylhydrazone, b.p. 100-103° C. This product also is referred to as 1-tert-butyl-2-isopropylidenehydrazide of benzoic acid.

Following the above procedure, but substituting p-chlorobenzoyl chloride for benzoyl chloride yields the product 1-tert-butyl-2-isopropylidenehydrazide of p-chlorobenzoic acid.

Substitution of the o-nitrobenzoyl chloride or o-fluorobenzoyl chloride for benzoyl chloride in the above procedure yields, respectively, 1-tert-butyl-2-isopropylidenehydrazide of o-nitrobenzoic acid and 1-tert-butyl-2-isopropylidenehydrazide of p-fluorobenzoic acid. The reactions are illustrated as follows:

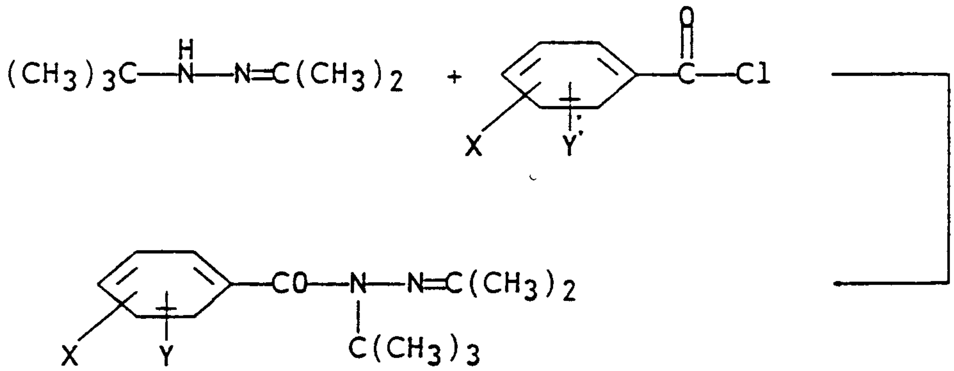

EXAMPLE 7

Preparation of benzoic acid, 1-tert-butylhydrazide

A solution of 0.5 g of the 1-tert-butyl-2-isopropylidenehydrazide of benzoic acid, 3 mL of 10% hydrochloric acid and 3 mL of methanol is mixed and allowed to stand for 12 hours. The mixture is made basic with dilute sodium hydroxide. The methanol is evaporated from the mixture yielding the product 1-tert-butylhydrazide of benzoic acid, m.p. 117-123 ° C.

The above procedure is used, but 1-tert-butyl-2-isopropylidenehydrazide of p-chlorobenzoic acid is substituted for 1-tert-butyl-2-isopropylidenehydrazide of benzoic acid. This yields 1-tert-butylhydrazide of p-chlorobenzoic acid, m.p. 134-136 ° C.

Similarly, 1-tert-butylhydrazide o-nitrobenzoic acid, m.p. 141-144 ° C and 1-tert-butylhydrazide of p-fluorobenzoic acid, m.p. 136-137 ° C; is prepared by the above reaction using the appropriately substituted benzoic acid, 1-tert-butyl-2-isopropylidenehydrazide. The reactions are illustrated as follows:

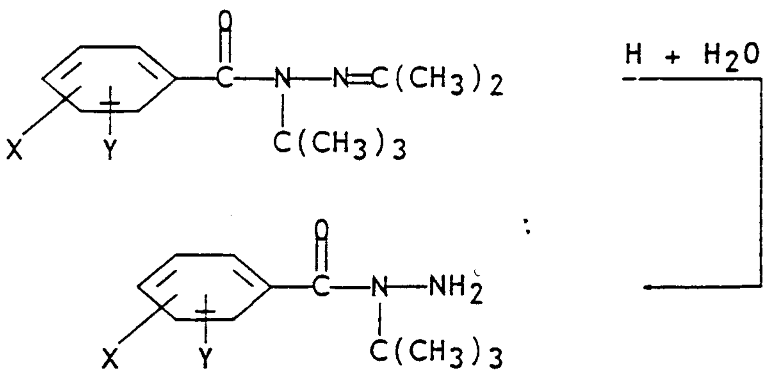


Insecticidal activity of the compounds of the invention

The compounds of the present invention exhibit insecticidal activity against a variety of insects at various concentrations of active ingredient in acetone-water solutions. As illustrative of this insecticidal activity is control of Spodoptera eridania (third-instar larvae, southern armyworm), Spodoptera eridania - (seven-day residual), Spodoptera eridania (third-instar cut-stem systemic test, southern armyworm), Anopheles quadrimaculatus (adults, common malaria mosquito), Heliothis virescens (third-instar tobacco budworm), Blattella germanica (residue test, adult male German cockroach), and Leptinotarsa decemlineata (Colorado potato beetles).

Further, systemic activity of the compounds is observed when tested for controlling Colorado potato beetles (Leptinotarsa decemlineata) on potato plants, (Solanum tuberosum). These beetles are resistant to carbamates, phosphates and pyrethroids but are controlled by the present compounds.

Bean plants, Phaseolus limensis, also are protected from southern armyworms, Spodoptera eridania and systemically protected from potato leafhoppers, Empoasca abrupta.

Maize plants (Zea mays L. plants) also are protected from insect attack of southern armyworm larvae (Spodoptera eridania, third-instar larvae, southern armyworm) and systemically from southern corn rootworm (Diabrotica undecimpunctata howardi).

Rice plants, Oryza sativa, are protected via systemic application, as well as by foliar application, from armyworms, Spodoptera frugiperda, and leafhoppers, Empoasca abrupta.

Cotton plants Gossypium hirsutum, also are systemically protected, as well as by foliar application from tobacco budworms (Heliotris virescens).

## Claims

### Claims for the following Contracting States : BE, CH, DE, FR, GB, IT, LI, LU, NL, SE

**1.** A compound having the structural formula (I)

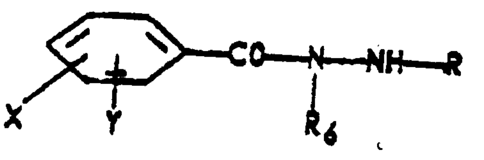

(I)

wherein R is hydrogen, $C_2$-$C_6$ alkyl or $C_5$-$C_6$ cycloalkyl, $R_6$ is tert-butyl; X and Y are each independently H, $C_1$-$C_3$ alkyl , $C_1$-$C_3$ alkoxy, $C_1$-$C_3$ alkylthio, $C_1$-$C_3$ alkyl-sulfinyl, $C_1$-$C_3$ alkylsulfonyl, cyano, F, Cl, Br, nitro, $CF_3$, $R_1CF_2Z$-, 1,1-difluoro-2,2-dichloroethoxy, $R_2CO$ or $R_3R_4N$; Z is $S(O)_n$ or O; $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl; $R_4$ is H, $C_1$-$C_3$ alkyl or $R_5CO$; $R_5$ is H or $C_1$-$C_3$ alkyl and n is 0, 1 or 2; with the provisos that when R is tert-butyl and Y is chloro in the para position of the ring, X is a substituent other than hydrogen, and when X and Y are both H, R is not hydrogen.

**2.** A compound according to claim 1, wherein R is hydrogen and $R_6$ is tert-butyl.

**3.** A compound according to Claim 1, wherein said compound is p-fluorobenzoic acid 1-tert-butylhydrazide.

**4.** An insecticidal composition characterizing: an insecticidally-effective amount of a compound of the formula II)

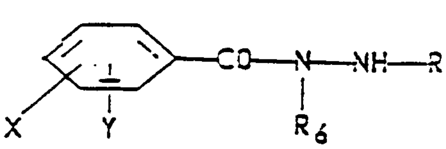

(II)

wherein R and $R_6$ are each independently hydrogen, $C_2$-$C_6$ alkyl or $C_5$-$C_6$ cycloalkyl; X and Y are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, I, nitro, $CF_3$, $R_1CF_2Z$-, 1,1-difluoro-2,2-dichloroethoxy, $R_2CO$ or $R_3R_4N$ and when taken together X and Y may form a ring in which XY are represented by the structure:

$-OCH_2O-$, $-OCF_2O-$ or 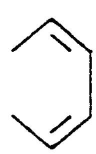 ;

Z is S(O)n or O; $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl; $R_4$ is H, $C_1$-$C_3$ alkyl or $R_5CO$; $R_5$ is H or $C_1$-$C_3$ alkyl and n is O, 1 or 2; with the provisos that when R is hydrogen, $R_6$ is $C_2$-$C_5$ alkyl or $C_5$-$C_6$ cycloalkyl and when $R_6$ is hydrogen, R is $C_2$-$C_5$ alkyl or $C_5$-$C_6$ cycloalkyl; a surfactant; and an inert solid or liquid diluent therefore.

**5.** A composition according to Claim 4 , wherein R is <u>tert</u>-butyl.

**6.** A method for protecting living plants through an extended period of active growth from insects which infest said living plants, said method characterizing: applying to the foliage of said plants or to the soil or other media in which they are growing, an insecticidally-effective amount of a compound with the formula,

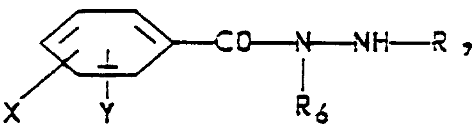

wherein R and $R_6$ are each independently hydrogen, $C_2$-$C_6$ alkyl or $C_5$-$C_6$ cycloalkyl; X and Y are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, I, nitro, $CF_3$, $R_1CF_2Z$-, 1,1-difluoro-2,2-dichloroethoxy, $R_2CO$ or $R_3R_4N$ and when taken together X and Y may form a ring in which XY are represented by the structure:

$-OCH_2O-$, $-OCF_2O-$ or 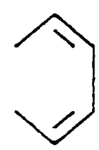 ;

Z is S(O)n or O; $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl; $R_4$ is H, $C_1$-$C_3$ alkyl or $R_5CO$; $R_5$ is H or $C_1$-$C_3$ alkyl and n is 0, 1 or 2; with the provisos that when R is hydrogen, $R_6$ is $C_2$-$C_5$ alkyl or $C_5$-$C_6$ cycloalkyl and when $R_6$ is hydrogen, R is $C_2$-$C_5$ alkyl or $C_5$-$C_6$ cycloalkyl.

**7.** A method according to Claim 6 , wherein said compound is applied to the foliage of plants or to the soil or other media in which they are growing at a rate of about 0.1 kg/ha to about 10.0 kg/ha.

**8.** A method according to Claim 6 , wherein said compound is applied to the foliage of said plants or to the soil or other media in which they are growing in the form of a dilute spray containing from about 10 ppm to 10,000 ppm of the said compound.

**9.** A method for systemically protecting plants from insect attack, said method characterizing: applying to the soil or other media in which said plants are growing, a systemically-effective insecticidal amount of a compound with formula,

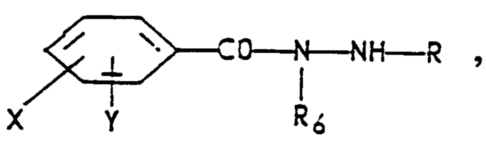

wherein R and $R_6$ are each independently hydrogen, $C_2$-$C_6$ alkyl or $C_5$-$C_6$ cycloalkyl; X and Y are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, I, nitro, $CF_3$, $R_1CF_2Z$-, 1,1-difluoro-2,2-dichloroethoxy, $R_2CO$ or $R_3R_4N$ and when taken together X and Y may form a ring in which XY are represented by the structure:

$-OCH_2O-$, $-OCF_2O-$ or 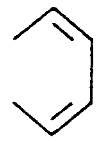 ;

Z is S(O)n or O; $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl; $R_4$ is H, $C_1$-$C_3$ alkyl or $R_5CO$; $R_5$ is H or $C_1$-$C_3$ alkyl and n is 0, 1 or 2; with the provisos that when R is hydrogen, $R_6$ is $C_2$-$C_5$ alkyl or $C_5$-$C_6$ cycloalkyl and when $R_6$ is hydrogen, R is $C_2$-$C_5$ alkyl or $C_5$-$C_6$ cycloalkyl.

**Claims for the following Contracting States : AT, ES, GR**

**1.** A process for preparing a compound having the structural formula:

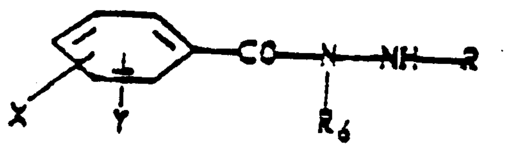

(I)

wherein R is hydrogen, $C_2$-$C_6$ alkyl or $C_5$-$C_6$ cycloalkyl, $R_6$ is tert-butyl; X and Y are each independently H, $C_1$-$C_3$ alkyl , $C_1$-$C_3$ alkoxy, $C_1$-$C_3$ alkylthio, $C_1$-$C_3$ alkyl-sulfinyl, $C_1$-$C_3$ alkylsulfonyl, cyano, F, Cl, Br, nitro, $CF_3$, $R_1CF_2Z$-, 1,1-difluoro-2,2-dichloroethoxy, $R_2CO$ or $R_3R_4N$; Z is $S(O)_n$ or O; $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl; $R_4$ is H, $C_1$-$C_3$alkyl or $R_5CO$; $R_5$ is H or $C_1$-$C_3$ alkyl and n is 0, 1 or 2; with the provisos that when R is tert-butyl and Y is chloro in the para position of the ring, X is a substituent other than hydrogen, and when X and Y are both N, R is not hydrogen, said process comprising reacting an alkyl- or cycloalkylhydrazide hydrohalide having the formula:

$RNHNH_2 \cdot HQ$

wherein Q is halogen and R is $C_2$-$C_6$ alkyl or $C_5$-$C_6$ cycloalkyl, with a benzoyl halide having the formula:

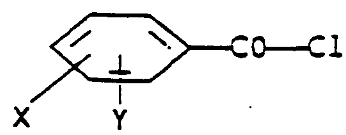

wherein X and Y are as defined above, in the presence of aqueous base; or said process comprises reacting a compound having the formula:

$R_6-NHNH=C(CH_3)_2$

wherein $R_6$ is tert-butyl with a benzoyl halide having the formula:

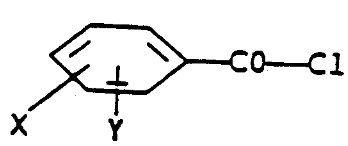

wherein X and Y are as defined above to yield a compound having the formula:

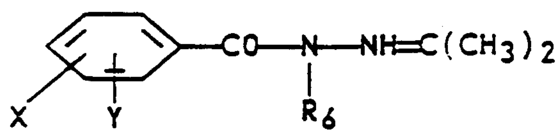

wherein X, Y and $R_6$ are as defined above and subjecting the thus-formed product to a hydrolysis with a dilute mineral acid in the presence of alcohol to obtain a compound having the formula:

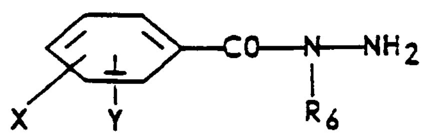

wherein $R_6$, X and Y are as defined above.

**2.** An insecticidal composition characterizing: an insecticidally-effective amount of a compound of the formula II):

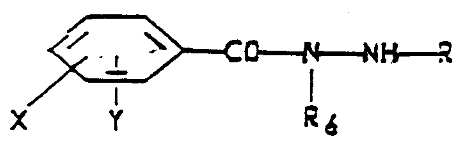 (II)

wherein R and $R_6$ are each independently hydrogen, $C_2$-$C_6$ alkyl or $C_5$-$C_6$ cycloalkyl; X and Y are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, I, nitro, $CF_3$, $R_1CF_2Z$-, 1,1-difluoro-2,2-dichloroethoxy, $R_2CO$ or $R_3R_4N$ and when taken together X and Y may form a ring in which XY are represented by the structure:

$-OCH_2O-$, $-OCF_2O-$ or 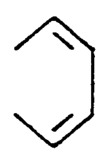 ;

Z is S(O)n or O; $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl; $R_4$ is H, $C_1$-$C_3$ alkyl or $R_5CO$; $R_5$ is H or $C_1$-$C_3$ alkyl and n is 0, 1 or 2; with the provisos that when R is hydrogen, $R_6$ is $C_2$-$C_5$ alkyl or $C_5$-$C_6$ cycloalkyl and when $R_6$ is hydrogen, R is $C_2$-$C_5$ alkyl or $C_5$-$C_6$ cycloalkyl; a surfactant; and an inert solid or liquid diluent therefore.

**3.** A composition according to Claim 2, wherein R is tert-butyl.

**4.** A method for protecting living plants through an extended period of active growth from insects which infest said living plants, said method characterizing: applying to the foliage of said planes or to the soil or other media in which they are growing, an insecticidally-effective amount of a compound with the formula,

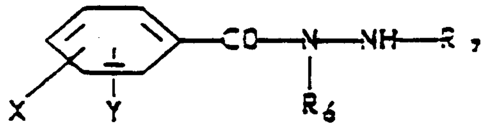

wherein R and $R_6$ are each independently hydrogen, $C_2$-$C_6$ alkyl or $C_5$-$C_6$ cycloalkyl; X and Y are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, I, nitro, $CF_3$, $R_1CF_2Z$-, 1,1-difluoro-2,2-dichloroethoxy, $R_2CO$ or $R_3R_4N$ and when taken together X and Y may form a ring in which XY are represented by the structure:

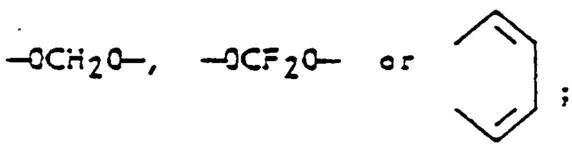

Z is S(O)n or O; $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl; $R_4$ is H, $C_1$-$C_3$ alkyl or $R_5CO$; $R_5$ is H or $C_1$-$C_3$ alkyl and n is 0, 1 or 2; with the provisos that when R is hydrogen, $R_6$ is $C_2$-$C_5$ alkyl or $C_5$-$C_6$ cycloalkyl and when $R_6$ is hydrogen, R is $C_2$-$C_5$ alkyl or $C_5$-$C_6$ cycloalkyl.

**5.** A method according to Claim 4 , wherein said compound is applied to the foliage of plants or to the soil or other media in which they are growing at a rate of about 0.1 kg/ha to about 10.0 kg/ha.

**6.** A method according to Claim 4 , wherein said compound is applied to the foliage of said plants or to the soil or other media in which they are growing in the form of a dilute spray containing from about 10 ppm to 10,000 ppm of the said compound.

**7.** A method for systemically protecting plants from insect attack, said method characterizing: applying to the soil or other media in which said plants are growing, a systemically-effective insecticidal amount of a compound with formula,

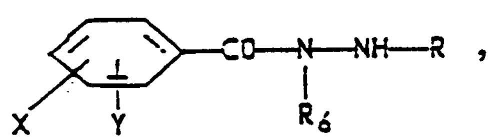

wherein R and $R_6$ are each independently hydrogen, $C_2$-$C_6$ alkyl or $C_5$-$C_6$ cycloalkyl; X and Y are each independently H, $C_1$-$C_3$ alkyl, $C_1$-$C_3$ alkoxy, $C_1$-$C_3$ alkylthio, $C_1$-$C_3$ alkylsulfinyl, $C_1$-$C_3$ alkylsulfonyl, cyano, F, Cl, Br, I, nitro, $CF_3$, $R_1CF_2Z$-, 1,1-difluoro-2,2-dichloroethoxy, $R_2CO$ or $R_3R_4N$ and when taken together X and Y may form a ring in which XY are represented by the structure:

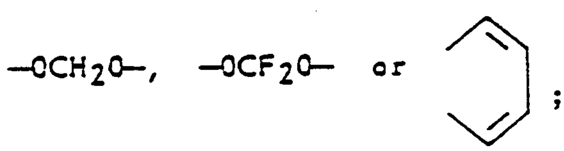

Z is S(O)n or O; $R_1$ is H, F, $CHF_2$, CHFCl or $CF_3$; $R_2$ is $C_1$-$C_3$ alkyl, $C_1$-$C_3$ alkoxy or $R_3R_4N$; $R_3$ is H or $C_1$-$C_3$ alkyl; $R_4$ is H, $C_1$-$C_3$ alkyl or $R_5CO$; $R_5$ is H or $C_1$-$C_3$ alkyl and n is 0, 1 or 2; with the provisos that when R is hydrogen, $R_6$ is $C_2$-$C_5$ alkyl or $C_5$-$C_5$ cycloalkyl and when $R_6$ is hydrogen, R is $C_2$-$C_5$

alkyl or $C_5$-$C_6$ cycloalkyl.

**Revendications**

**Revendications pour les Etats contractants suivants : BE, CH, DE, FR, GB, IT, LI, LU, NL, SE**

**1.** Composé de formule structurale (I):

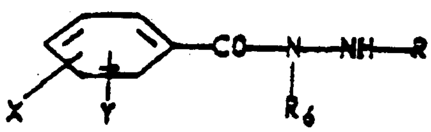

(I)

dans laquelle R représente l'hydrogène, un alkyle en $C_2$-$C_6$ ou un cycloalkyle en $C_5$-$C_6$, $R_6$ est le *tert*-butyle; X et Y représentent chacun séparément l'atome d'hydrogène, un alkyle en $C_1$-$C_3$, un alkoxy en $C_1$-$C_3$, un alkylthio en $C_1$-$C_3$, un alkyl-sulfinyle en $C_1$-$C_3$, un alkylsulfonyle en $C_1$-$C_3$, le groupe cyano, l'atome de fluor, de chlore, de brome, le groupe nitro, $CF_3$, $R_1CF_2Z$-, 1,1-difluoro-2,2-dichloroéthoxy, $R_2CO$ ou $R_3R_4N$; Z représente $S(O)_n$ ou O; $R_1$ représente H, F, $CHF_2$ CHFCl ou $CF_3$; $R_2$ représente un alkyle en $C_1$-$C_3$, un alkoxy en $C_1$-$C_3$ ou $R_3R_4N$; $R_3$ représente H ou un alkyle en $C_1$-$C_3$; $R_4$ représente H, un alkyle en $C_1$-$C_3$ ou $R_5CO$; $R_5$ représente H ou un alkyle en $C_1$-$C_3$ et n représente 0, 1 ou 2; à condition que lorsque R est le *tert*-butyle et que Y est un radical chloro en position para du cycle, X soit un substituant autre que l'hydrogène, et que lorsque X et Y représentent tous deux l'atome d'hydrogène, R ne soit pas l'atome d'hydrogène.

**2.** Composé selon la Revendication 1, dans lequel R est l'atome d'hydrogène et $R_6$ est le *tert*-butyle.

**3.** Composé selon la Revendication 1, dans lequel ledit composé est le 1-*tert*-butylhydrazide de l'acide *p*-fluorobenzoïque.

**4.** Composition insecticide comprenant: une quantité efficace du point de vue insecticide d'un composé de formule (II):

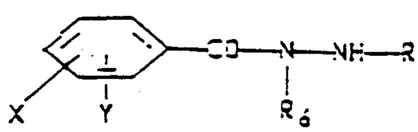

(II)

dans laquelle R et $R_6$ représentent chacun indépendamment l'atome d'hydrogène, un alkyle en $C_2$-$C_6$ ou un cycloalkyle en $C_5$-$C_6$; X et Y représentent chacun séparément l'atome d'hydrogène, un alkyle en $C_1$-$C_3$, un alkoxy en $C_1$-$C_3$, un alkylthio en $C_1$-$C_3$, un alkylsulfinyle en $C_1$-$C_3$, un alkylsulfonyle en $C_1$-$C_3$, le groupe cyano, l'atome de fluor, de chlore, de brome, d'iode, le groupe nitro, $CF_3$, $R_1CF_3Z$-, 1,1-difluoro-2,2-dichloroéthoxy, $R_2CO$ ou $R_3R_4N$ et, lorsqu'ils sont pris ensemble, X et Y peuvent former un cycle dans lequel XY est représenté par la structure:

$-OCH_2O-$, $-OCF_2O-$ ou 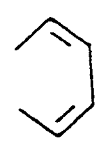 ;

Z représente $S(O)_n$ ou O; $R_1$ représente H, F, $CHF_2$, CHFCl ou $CF_3$; $R_2$ représente un alkyle en $C_1$-$C_3$, un alkoxy en $C_1$-$C_3$ ou $R_3R_4N$; $R_3$ représente H ou un alkyle en $C_1$-$C_3$; $R_4$ représente H, un alkyle en $C_1$-$C_3$ ou $R_5CO$; $R_5$ représente H ou un alkyle en $C_1$-$C_3$; et n représente 0, 1 ou 2, à condition que lorsque R est l'atome d'hydrogène, $R_6$ représente un alkyle en $C_2$-$C_5$ ou un cycloalkyle en $C_5$-$C_6$, et que lorsque $R_6$ est l'atome d'hydrogène, R représente un alkyle en $C_2$-$C_5$ ou un cycloalkyle en $C_5$-$C_6$; un surfactant; et un solide inerte ou un diluant liquide approprié.

**5.** Composition selon la Revendication 4, dans laquelle R est le <u>tert</u>-butyle.

**6.** Méthode de protection des végétaux vivants pendant une période croissance active prolongée contre les insectes qui infestent lesdits végétaux vivants, ladite méthode comprenant: l'application sur le feuillage desdits végétaux ou sur le sol ou d'autres milieux dans lesquels ils croissent, d'une quantité efficace du point de vue insecticide d'un composé de formule:

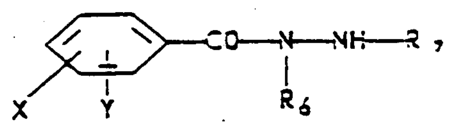

dans laquelle R et $R_6$ représentent chacun indépendamment l'atome d'hydrogène, un alkyle en $C_2$-$C_6$ ou un cycloalkyle en $C_5$-$C_6$; X et Y représentent chacun séparément l'atome d'hydrogène, un alkyle en $C_1$-$C_3$, un alkoxy en $C_1$-$C_3$, un alkylthio en $C_1$-$C_3$, un alkylsulfinyle en $C_1$-$C_3$, un alkylsulfonyle en $C_1$-$C_3$, le groupe cyano, l'atome de fluor, de chlore, de brome, d'iode, le groupe nitro, $CF_3$, $R_1CF_2Z$-, 1,1-difluoro-2,2-dichloroéthoxy, $R_2CO$ ou $R_3R_4N$ et, lorsqu'ils sont pris ensemble, X et Y peuvent former un cycle dans lequel XY est représenté par la structure:

**$-OCH_2O-$, $-OCF_2O-$ ou** 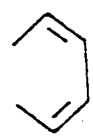 **;**

Z représente $S(O)_n$ ou O; $R_1$ représente H, F, $CHF_2$, CHFCl ou $CF_3$; $R_2$ représente un alkyle en $C_1$-$C_3$, un alkoxy en $C_1$-$C_3$ ou $R_3R_4N$; $R_3$ représente H ou un alkyle en $C_1$-$C_3$; $R_4$ représente H, un alkyle en $C_1$-$C_3$ ou $R_5CO$; $R_5$ représente H ou un alkyle en $C_1$-$C_3$; et n représente 0, 1 ou 2, à condition que lorsque R est l'atome d'hydrogène, $R_6$ représente un alkyle en $C_2$-$C_5$ ou un cycloalkyle en $C_5$-$C_6$, et que lorsque $R_6$ est l'atome d'hydrogène, R représente un alkyle en $C_2$-$C_5$ ou un cycloalkyle en $C_5$-$C_6$.

**7.** Méthode selon la Revendication 6, dans laquelle ledit composé est appliqué sur le feuillage des plantes ou sur le sol ou d'autres milieux dans lesquels elles croissent à une concentration comprise entre 0,1 kg/hectare environ et 10,0 kg/hectare environ.

**8.** Méthode selon la Revendication 6, dans laquelle ledit composé est appliqué sur le feuillage desdites plantes ou sur le sol ou d'autres milieux dans lesquels elles croissent sous la forme d'une solution diluée à pulvériser contenant entre 10 ppm environ et 10.000 ppm environ dudit composé.

**9.** Méthode pour la protection systémique des plantes contre l'attaque des insectes, ladite méthode comprenant: l'application sur le sol ou à d'autres milieux dans lesquels croissent lesdites plantes d'une quantité efficace du point de vue insecticide d'un composé de formule:

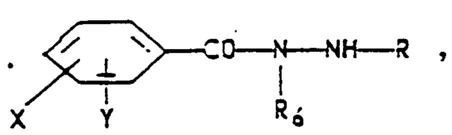

dans laquelle R et $R_6$ représentent chacun indépendamment l'atome d'hydrogène, un alkyle en $C_2$-$C_6$ ou un cycloalkyle en $C_5$-$C_6$; X et Y représentent chacun séparément l'atome d'hydrogène, un alkyle en $C_1$-$C_3$, un alkoxy en $C_1$-$C_3$, un alkylthio en $C_1$-$C_3$, un alkylsulfinyle en $C_1$-$C_3$, un alkylsulfonyle en $C_1$-$C_3$, le groupe cyano, l'atome de fluor, de chlore, de brome, d'iode, le groupe nitro, $CF_3$, $R_1CF_2Z$-, 1,1-difluoro-2,2-dichloroéthoxy, $R_2CO$ ou $R_3R_4N$ et, lorsqu'ils sont pris ensemble, X et Y peuvent former un cycle dans lequel XY est représenté par la structure:

$-OCH_2O-$, $-OCF_2O-$ ou 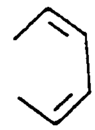 ;

Z représente $S(O)_n$ ou O; $R_1$ représente H, F, $CHF_2$, CHFCl ou $CF_3$; $R_2$ représente un alkyle en $C_1$-$C_3$, un alkoxy en $C_1$-$C_3$ ou $R_3R_4N$; $R_3$ représente H ou un alkyle en $C_1$-$C_3$; $R_4$ représente H, un alkyle en $C_1$-$C_3$ ou $R_5CO$; $R_5$ représente H ou un alkyle en $C_1$-$C_3$; et n représente 0, 1 ou 2, à condition que lorsque R est l'atome d'hydrogène, $R_6$ représente un alkyle en $C_2$-$C_5$ ou un cycloalkyle en $C_5$-$C_6$, et que lorsque $R_6$ est l'atome d'hydrogène, R représente un alkyle en $C_2$-$C_5$ ou un cycloalkyle en $C_5$-$C_6$.

**Revendications pour les Etats contractants suivants : AT, ES, GR**

**1.** Un procédé de préparation d'un composé de formule structurale (I) :

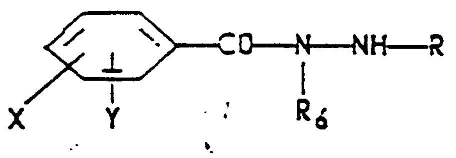 , (I)

dans laquelle R représente l'hydrogène, un alkyle en $C_2$-$C_6$ ou un cycloalkyle en $C_5$-$C_6$, $R_6$ est le tert-butyle; X et Y représentent chacun séparément l'atome d'hydrogène, un alkyle en $C_1$-$C_3$, un alkoxy en $C_1$-$C_3$, un alkylthio en $C_1$-$C_3$, un alkyl-sulfinyle en $C_1$-$C_3$, un alkylsulfonyle en $C_1$-$C_3$, le groupe cyano, l'atome de fluor, de chlore, de brome, le groupe nitro, $CF_3$, $R_1CF_2Z$-, 1,1-difluoro-2,2-dichloroéthoxy, $R_2CO$ ou $R_3R_4N$; Z représente $S(O)_n$ ou O; $R_1$ représente H, F, $CHF_2$, CHFCl ou $CF_3$; $R_2$ représente un alkyle en $C_1$-$C_3$, un alkoxy en $C_1$-$C_3$ ou $R_3R_4N$; $R_3$ représente H ou un alkyle en $C_1$-$C_3$; $R_4$ représente H, un alkyle en $C_1$-$C_3$ ou $R_5CO$; $R_5$ représente H ou un alkyle en $C_1$-$C_3$ et n représente 0, 1 ou 2; à condition que lorsque R est le tert-butyle et que Y est un radical chloro en position para du cycle, X soit un substituant autre que l'hydrogène, et que lorsque X et Y représentent tous deux l'atome d'hydrogène, R ne soit pas l'atome d'hydrogène,
ledit procédé comprenant la réaction d'un halohydrate d'alkyl- ou cycloalkyl-hydrazide de formule :

$RNHNH_2.HQ$

dans laquelle Q représente un halogène et R représente un alkyle en $C_2$-$C_6$ ou un cycloalkyle en $C_5$-$C_6$,
avec un halogénure de benzoyle ayant la formule :

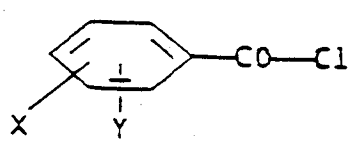

dans laquelle X et Y sont tels que définis ci-dessus, en présence d'une base aqueuse; ou ledit procédé comprend la réaction d'un composé ayant la formule :

$R_6\ NHNH = C(CH_3)_2$

dans laquelle $R_6$ représente le tertio-butyle avec un halogénure ayant la formule :

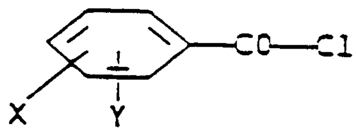

dans laquelle X et Y sont tels que définis ci-dessus pour obtenir un composé ayant la formule :

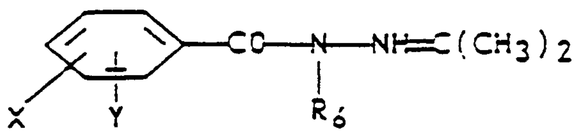

dans laquelle X, Y et $R_6$ sont tels que définis ci-dessus et en soumettant le produit ainsi formé à une hydrolyse à l'aide d'un acide minéral dilué en présence d'un alcool pour obtenir u n composé ayant la formule :

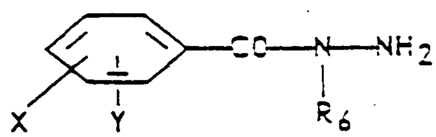

dans laquelle $R_6$, X et Y sont tels que définis ci-dessus.

**2.** Composition insecticide comprenant : une quantité efficace du point de vue insecticide d'un composé de formule (II) :

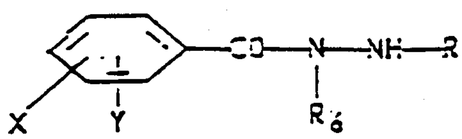

(II)

dans laquelle R et $R_6$ représentent chacun indépendamment l'atome d'hydrogène, un alkyle en $C_2$-$C_6$ ou un cycloalkyle en $C_5$-$C_6$; X et Y représentent chacun séparément l'atome d'hydrogène, un alkyle en $C_1$-$C_3$, un alkoxy en $C_1$-$C_3$, un alkylthio en $C_1$-$C_3$, un alkylsulfinyle en $C_1$-$C_3$, un alkylsulfonyle en $C_1$-$C_3$, le groupe cyano, l'atome de fluor, de chlore, de brome, d'iode, le groupe nitro, $CF_3$, $R_1CF_2Z$-, 1,1-difluoro-2,2'dichloroéthoxy, $R_2CO$ ou $R_3R_4N$ et, lorsqu'ils sont pris ensemble, X et Y peuvent former un cycle dans lequel XY est représenté par la structure:

$-OCH_2O-$, $-OCF_2O-$ ou 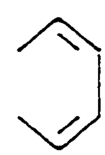 ;

Z représente $S(O)_n$ ou O; $R_1$ représente H, F, $CHF_2$, CHFCl ou $CF_3$; $R_2$ représente un alkyle en $C_1$-$C_3$, un alkoxy en $C_1$-$C_3$ ou $R_3R_4N$; $R_3$ représente H ou un alkyle en $C_1$-$C_3$; $R_4$ représente H, un alkyle en $C_1$-$C_3$ ou $R_5CO$; $R_5$ représente H ou un alkyle en $C_1$-$C_3$; et n représente 0, 1 ou 2, à condition que lorsque R est l'atome d'hydrogène, $R_6$ représente un alkyle en $C_2$-$C_5$ ou un cycloalkyle en $C_5$-$C_6$, et que lorsque $R_6$ est l'atome d'hydrogène, R représente un alkyle en $C_2$-$C_5$ ou un cycloalkyle en $C_5$-$C_6$; un surfactant; et un solide inerte ou un diluant liquide approprié.

**3.** Composition selon la revendication 2, dans laquelle R représente le tertio-butyle.

**4.** Méthode de protection des végétaux vivants pendant une période de croissance active prolongée

contre les insectes qui infestent lesdits végétaux vivants, ladite méthode comprenant :
l'application sur le feuillage desdits végétaux ou sur le sol ou d'autres milieux dans lesquels ils croissent, d'une quantité efficace du point de vue insecticide d'un composé de formule:

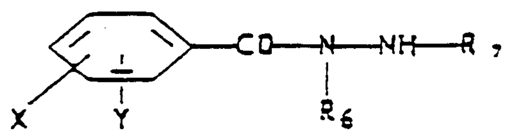

dans laquelle R et $R_6$ représentent chacun indépendamment l'atome d'hydrogène, un alkyle en $C_2$-$C_6$ ou un cycloalkyle en $C_5$-$C_6$; X et Y représentent chacun séparément l'atome d'hydrogène, un alkyle en $C_1$-$C_3$, un alkoxy en $C_1$-$C_3$, un alkylthio en $C_1$-$C_3$, un alkylsulfinyle en $C_1$-$C_3$, un alkylsulfonyle en $C_1$-$C_3$, le groupe cyano, l'atome de fluor, de chlore, de brome, d'iode, le groupe nitro, $CF_3$, $R_1CF_2Z$-, 1,1-difluoro-2,2-dichloroéthoxy, $R_2CO$ ou $R_3R_4N$ et, lorsqu'ils sont pris ensemble, X et Y peuvent former un cycle dans lequel XY est représenté par la structure:

$-OCH_2O-$, $-OCF_2O-$ ou 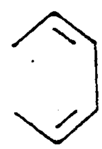 ;

Z représente $S(O)_n$ ou O; $R_1$ représente H, F, $CHF_2$, CHFCl ou $CF_3$; $R_2$ représente un alkyle en $C_1$-$C_3$, un alkoxy en $C_1$-$C_3$ ou $R_3R_4N$; $R_3$ représente H ou un alkyle en $C_1$-$C_3$; $R_4$ représente H, un alkyle en $C_1$-$C_3$ ou $R_5CO$; $R_5$ représente H ou un alkyle en $C_1$-$C_3$; et n représente 0, 1 ou 2, à condition que lorsque R est l'atome d'hydrogène, $R_6$ représente un alkyle en $C_2$-$C_5$ ou un cycloalkyle en $C_5$-$C_6$, et que lorsque $R_6$ est l'atome d'hydrogène, R représente un alkyle en $C_2$-$C_5$ ou un cycloalkyle en $C_5$-$C_6$.

**5.** Méthode selon la revendication 4, dans laquelle ledit composé est appliqué sur le feuillage des plantes ou sur le sol ou d'autres milieux dans lesquels elles croissent à une concentration comprise entre 0,1 kg/hectare environ et 10,0 kg/hectare environ.

**6.** Méthode selon la revendication 4, dans laquelle ledit composé est appliqué sur le feuillage desdites plantes ou sur le sol ou d'autres milieux dans lesquels elles croissent sous la forme d'une solution diluée à pulvériser contenant entre 10 ppm environ à 10.000 ppm environ dudit composé.

**7.** Méthode pour la protection systémique des plantes contre l'attaque des insectes, ladite méthode comprenant : l'application sur le sol ou à d'autres milieux dans lesquels croissent lesdites plantes d'une quantité efficace du point de vue insecticide d'un composé de formule :

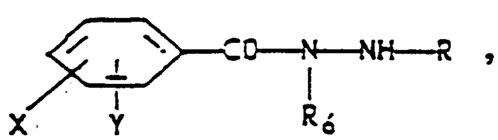

dans laquelle R et $R_6$ représentent chacun indépendamment l'atome d'hydrogène, un alkyle en $C_2$-$C_6$ ou un cycloalkyle en $C_5$-$C_6$; X et Y représentent chacun séparément l'atome d'hydrogène, un alkyle en $C_1$-$C_3$, un alkoxy en $C_1$-$C_3$, un alkylthio en $C_1$-$C_3$, un alkylsulfinyle en $C_1$-$C_3$, un alkylsulfonyle en $C_1$-$C_3$, le groupe cyano, l'atome de fluor, de chlore, de brome, d'iode, le groupe nitro, $CF_3$, $R_1CF_2Z$-, 1,1-difluoro-2,2-dichloroéthoxy, $R_2CO$ ou $R_3R_4N$ et, lorsqu'ils sont pris ensemble, X et Y peuvent former un cycle dans lequel XY est représenté par la structure:

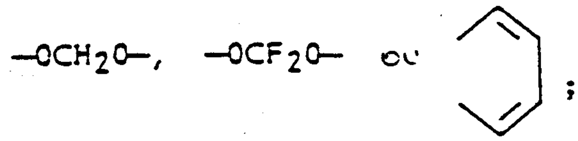

Z représente $S(O)_n$ ou O; $R_1$ représente H, F, $CHF_2$, CHFCl ou $CF_3$; $R_2$ représente un alkyle en $C_1$-$C_3$, un alkoxy en $C_1$-$C_3$ ou $R_3R_4N$; $R_3$ représente H ou un alkyle en $C_1$-$C_3$; $R_4$ représente H, un alkyle en $C_1$-$C_3$ ou $R_5CO$; $R_5$ représente H ou un alkyle en $C_1$-$C_3$; et n représente 0, 1 ou 2, à condition que lorsque R est l'atome d'hydrogène, $R_6$ représente un alkyle en $C_2$-$C_5$ ou un cycloalkyle en $C_5$-$C_6$, et que lorsque $R_6$ est l'atome d'hydrogène, R représente un alkyle en $C_2$-$C_5$ ou un cycloalkyle en $C_5$-$C_6$.

## Patentansprüche

## Patentansprüche für folgende Vertragsstaaten : BE, CH, DE, FR, GB, IT, LI, LU, NL, SE

**1.** Verbindung mit der Strukturformel (I):

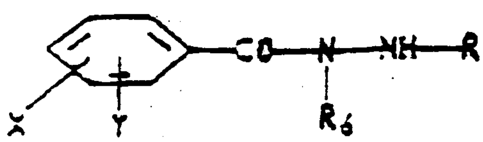

(I)

wobei R für Wasserstoff, $C_{2-6}$-Alkyl oder $C_{5-6}$-Cycloalkyl steht, $R_6$ für tert-Butyl steht; X und Y jeweils unabhängig H, $C_{1-3}$-Alkyl, $C_{1-3}$-Alkoxy, $C_{1-3}$-Alkylthio, $C_{1-3}$-Alkylsulfinyl, $C_{1-3}$-Alkylsulfonyl, Cyano, F, Cl, Br, Nitro, $CF_3$, $R_1CF_2Z$-, 1,1-Difluor-2,2-dichlorethoxy, $R_2CO$ oder $R_3R_4N$ stehen; Z für $S(O)_n$ oder O steht; $R_1$ für H, F, $CHF_2$, CHFCl oder $CF_3$ steht; $R_2$ für $C_{1-3}$-Alkyl, $C_{1-3}$-Alkoxy oder $R_3R_4N$ steht; $R_3$ für H oder $C_{1-3}$-Alkyl steht; $R_4$ für H, $C_{1-3}$-Alkyl oder $R_5CO$ steht; $R_5$ für H oder $C_{1-3}$-Alkyl steht und n für 0, 1 oder 2 steht; mit den Maßgaben, daß dann, wenn R tert-Butyl ist und Y für Chlor in der Para-Position des Rings steht, X ein Substituent ist, der nicht Wasserstoff ist, und daß dann, wenn X und Y beide H sind, R nicht für Wasserstoff steht.

**2.** Verbindung gemäß Anspruch 1, wobei R für Wasserstoff steht und $R_6$ für tert-Butyl steht.

**3.** Verbindung gemäß Anspruch 1, wobei die Verbindung p-Fluorbenzosäure-1-tert-Butylhydrazid ist.

**4.** Insektizide Zusammensetzung, gekennzeichnet durch eine insektizid wirksame Menge einer Verbindung der Formel (II)

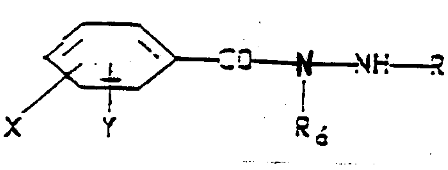

(II)

wobei R und $R_6$ jeweils unabhängig für Wasserstoff, $C_{2-6}$-Alkyl oder $C_{5-6}$-Cycloalkyl stehen; X und Y jeweils unabhängig für H, $C_{1-3}$-Alkyl, $C_{1-3}$-Alkoxy, $C_{1-3}$-Alkylthio, $C_{1-3}$-Alkylsulfinyl, $C_{1-3}$-Alkylsulfonyl, Cyano, F, Cl, Br, J, Nitro, $CF_3$, $R_1CF_2Z$-, 1,1-Difluor-2,2-dichlorethoxy, $R_2CO$ oder $R_3R_4N$ stehen und falls sie zusammengefaßt sind, X und Y einen Ring bilden können, in dem XY die folgende Struktur darstellt:

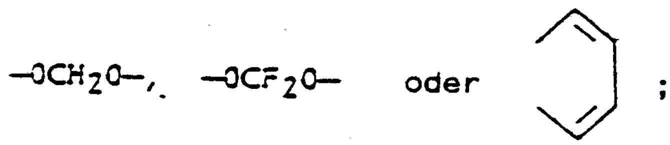

Z für $S(O)_n$ oder O steht; $R_1$ für H, F, $CHF_2$, CHFCl oder $CF_3$ steht; $R_2$ für $C_{1-3}$-Alkyl, $C_{1-3}$-Alkoxy oder $R_3R_4N$ steht; $R_3$ für H oder $C_{1-3}$-Akyl steht; $R_4$ für H, $C_{1-3}$-Alkyl oder $R_5CO$ steht; $R_5$ für H oder $C_{1-3}$-Alkyl steht und n für 0, 1 oder 2 steht; mit den Maßgaben, daß dann, wenn R für H steht, $R_6$ für $C_{2-5}$-Alkyl oder $C_{5-6}$-Cycloalkyl steht, und daß dann, wenn $R_6$ für Wasserstoff steht, R für $C_{2-5}$-Alkyl oder $C_{5-6}$-Alkyl steht; ein Surfaktans; und ein inertes, festes oder flüssiges Verdünnungsmittel dafür.

**5.** Zusammensetzung gemäß Anspruch 4, wobei R für tert-Butyl steht.

**6.** Verfahren zum Schutz von lebenden Pflanzen über einen längeren Zeitraum des aktiven Wachstums vor Insekten, welche die lebenden Pflanzen befallen, wobei das Verfahren dadurch gekennzeichnet ist, das man auf das Blattwerk der Pflanzen oder auf den Boden oder andere Medien, in denen sie wachsen, eine insektizid wirksame Menge einer Verbindung der folgenden Formel appliziert

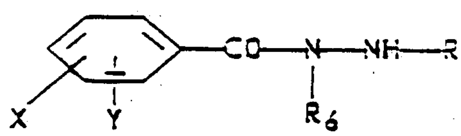

wobei R und $R_6$ jeweils unabhängig für Wasserstoff, $C_{2-6}$-Alkyl oder $C_{5-6}$-Cycloalkyl stehen; X und Y jeweils unabhängig für H, $C_{1-3}$-Alkyl, $C_{1-3}$-Alkoxy, $C_{1-3}$-Alkylthio, $C_{1-3}$-Alkylsulfinyl, $C_{1-3}$-Alkylsulfonyl, Cyano, F, Cl, Br, J, Nitro, $CF_3$, $R_1CF_2Z$-, 1,1-Difluor-2,2-dichlorethoxy, $R_2CO$ oder $R_3R_4N$ stehen und falls sie zusammengefaßt sind, X und Y einen Ring bilden können, in dem XY die folgende Struktur darstellt:

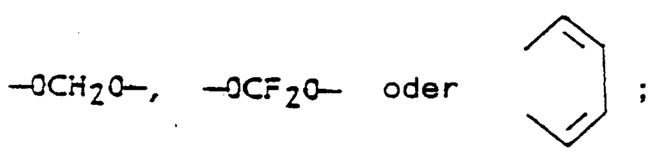

Z für $S(O)_n$ oder O steht; $R_1$ für H, F, $CHF_2$, CHFCl oder $CF_3$ steht; $R_2$ für $C_{1-3}$-Alkyl, $C_{1-3}$-Alkoxy oder $R_3R_4N$ steht; $R_3$ für H oder $C_{1-3}$-Akyl steht; $R_4$ für H, $C_{1-3}$-Alkyl oder $R_5CO$ steht; $R_5$ für H oder $C_{1-3}$-Alkyl steht und n für 0, 1 oder 2 steht; mit den Maßgaben, daß dann, wenn R für H steht, $R_6$ für $C_{2-5}$-Alkyl oder $C_{5-6}$-Cycloalkyl steht, und daß dann, wenn $R_6$ für Wasserstoff steht, R für $C_{2-5}$-Alkyl oder $C_{5-6}$-Alkyl steht.

**7.** Verfahren gemäß Anspruch 6, wobei die Verbindung auf das Blattwerk der Pflanzen oder auf den Boden oder andere Medien in denen diese wachesen mit einer Rate von etwa 0,1 kg pro ha bis etwa 10,0 kg pro ha appliziert wird.

**8.** Verfahren gemäß Anspruch 6, wobei die Verbindung auf das Blattwerk der Pflanzen oder auf den Boden oder andere Medien, in denen sie wachsen, in der Form eines verdünnten Sprays, enthaltend etwa 10 ppm bis 10 000 ppm der Verbindung appliziert wird.

**9.** Verfahren, zum systemischen Schutz von Pflanzen vor Insektenbefall, wobei das Verfahren dadurch gekennzeichnet ist, daß man auf den Boden oder andere Medien, in denen die Pflanzen wachsen, eine systemisch wirksame insektizide Menge einer Verbindung der folgenden Formel appliziert

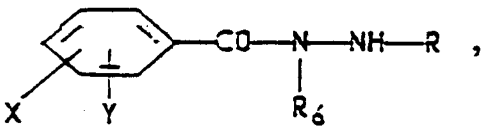

wobei R und $R_6$ jeweils unabhängig für Wasserstoff, $C_{2-6}$-Alkyl oder $C_{5-6}$-Cycloalkyl stehen; X und Y jeweils unabhängig für H, $C_{1-3}$-Alkyl, $C_{1-3}$-Alkoxy, $C_{1-3}$-Alkylthio, $C_{1-3}$-Alkylsulfinyl, $C_{1-3}$-Alkylsulfonyl, Cyano, F, Cl, Br, J, Nitro, $CF_3$, $R_1CF_2Z$-, 1,1-Difluor-2,2-dichlorethoxy, $R_2CO$ oder $R_3R_4N$ stehen und falls sie zusammengefaßt sind, X und Y einen Ring bilden können, in dem XY die folgende Struktur darstellt:

$-OCH_2O-$, $-OCF_2O-$ oder 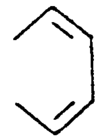 ;

Z für $S(O)_n$ oder O steht; $R_1$ für H, F, $CHF_2$, CHFCl oder $CF_3$ steht; $R_2$ für $C_{1-3}$-Alkyl, $C_{1-3}$-Alkoxy oder $R_3R_4N$ steht; $R_3$ für H oder $C_{1-3}$-Akyl steht; $R_4$ für H, $C_{1-3}$-Alkyl oder $R_5CO$ steht; $R_5$ für H oder $C_{1-3}$-Alkyl steht und n für 0, 1 oder 2 steht; mit den Maßgaben, daß dann, wenn R für H steht, $R_6$ für $C_{2-5}$-Alkyl oder $C_{5-6}$-Cycloalkyl steht, und daß dann, wenn $R_6$ für Wasserstoff steht, R für $C_{2-5}$-Alkyl oder $C_{5-6}$-Cycloalkyl steht.

**Patentansprüche für folgende Vertragsstaaten : AT, ES, GR**

**1.** Verfahren zur Herstellung einer Verbindung mit der Strukturformel

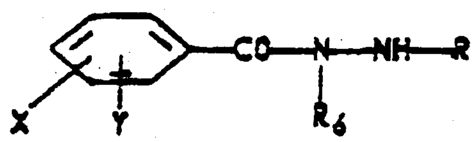

(I)

wobei R für Wasserstoff, $C_{2-6}$-Alkyl oder $C_{5-6}$-Cycloalkyl steht, $R_6$ für tert-Butyl steht; X und Y jeweils unabhängig H, $C_{1-3}$-Alkyl, $C_{1-3}$-Alkoxy, $C_{1-3}$-Alkylthio, $C_{1-3}$-Alkylsulfinyl, $C_{1-3}$-Alkylsulfonyl, Cyano, F, Cl, Br, Nitro, $CF_3$, $R_1CF_2Z$-, 1,1-Difluor-2,2-dichlorethoxy, $R_3CO$ oder $R_3R_4N$ stehen; Z für $S(O)_n$ oder O steht; $R_1$ für H, F, $CHF_2$, CHFCl oder $CF_3$ steht; $R_2$ für $C_{1-3}$-Alkyl, $C_{1-3}$-Alkoxy oder $R_3R_4N$ steht; $R_3$ für H oder $C_{1-3}$-Alkyl steht; $R_4$ für H, $C_{1-3}$-Alkyl oder $R_5CO$ steht; $R_5$ für H oder $C_{1-3}$-Alkyl steht und n für 0, 1 oder 2 steht; mit den Maßgaben, daß dann, wenn R tert-Butyl ist und Y für Chlor in der Para-Position des Rings steht, X ein Substituent ist, der nicht Wasserstoff ist, und daß dann, wenn X und Y beide H sind, R nicht für Wasserstoff steht, wobei das Verfahren die Umsetzung eines Akyl- oder Cycloalkylhydrazidhydrohalogenids der folgenden Formel:

$RNHNH_2 \cdot HQ$

wobei Q für Halogen steht und R für $C_{2-6}$-Alkyl oder $C_{5-6}$-Cycloalkyl steht, mit einem Benzoylhalogenid der Formel:

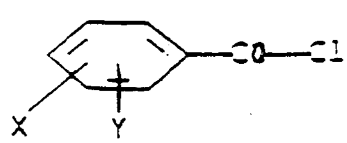

wobei X und Y wie oben definiert sind, in Anwesenheit einer wässrigen Base umfaßt; oder wobei das Verfahren die Umsetzung einer Verbindung der Formel:

$R_6-NHNH=C(CH_3)_2$

wobei $R_6$ für tert-Butyl steht, mit einem Benzoylhalogenid der Formel:

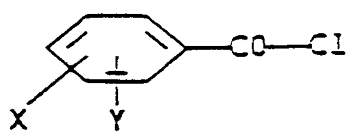

wobei X und Y wie oben definiert sind, umfaßt, um eine Verbindung mit der folgenden Formel zu erhalten:

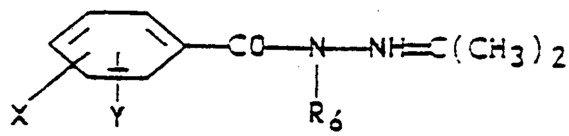

wobei X, Y und $R_6$ wie oben definiert sind, und das so gebildete Produkt einer Hydrolyse unterwirft mit einer verdünnten Mineralsäure in Anwesenheit von Alkohol, um eine Verbindung der folgenden Formel zu erhalten:

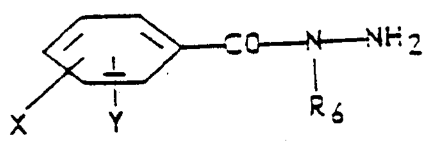

wobei $R_6$, X und Y wie oben definiert sind.

**2.** Insektizide Zusammensetzung, gekennzeichnet durch eine insektizid wirksame Menge einer Verbindung der Formel (II)

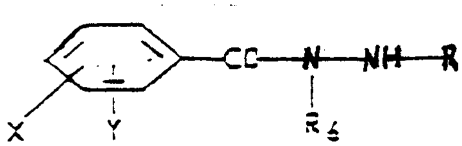 (II)

wobei R und $R_6$ jeweils unabhängig für Wasserstoff, $C_{2-6}$-Alkyl oder $C_{5-6}$-Cycloalkyl stehen; X und Y jeweils unabhängig für H, $C_{1-3}$-Alkyl, $C_{1-3}$-Alkoxy, $C_{1-3}$-Alkylthio, $C_{1-3}$-Alkylsulfinyl, $C_{1-3}$-Alkylsulfonyl, Cyano, F, Cl, Br, J, Nitro, $CF_3$, $R_1CF_2Z$-, 1,1-Difluor-2,2-dichlorethoxy, $R_2CO$ oder $R_3R_4N$ stehen und falls sie zusammengefaßt sind, X und Y einen Ring bilden können, in dem XY die folgende Struktur darstellt:

$-OCH_2O-$, $-OCF_2O-$ oder 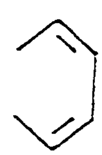 ;

Z für $S(O)_n$ oder O steht; $R_1$ für H, F, $CHF_2$, CHFCl oder $CF_3$ steht; $R_2$ für $C_{1-3}$-Alkyl, $C_{1-3}$-Alkoxy oder $R_3R_4N$ steht; $R_3$ für H oder $C_{1-3}$-Akyl steht; $R_4$ für H, $C_{1-3}$-Alkyl oder $R_5CO$ steht; $R_5$ für H oder $C_{1-3}$-Alkyl steht und n für 0, 1 oder 2 steht; mit den Maßgaben, daß dann, wenn R für H steht, $R_6$ für $C_{2-5}$-Alkyl oder $C_{5-6}$-Cycloalkyl steht, und daß dann, wenn $R_6$ für Wasserstoff steht, R für $C_{2-5}$-Alkyl oder $C_{5-6}$-Alkyl steht; ein Surfaktans; und ein inertes, festes oder flüssiges Verdünnungsmittel dafür.

**3.** Zusammensetzung gemäß Anspruch 2, wobei R für tert-Butyl steht.

**4.** Verfahren zum Schutz von lebenden Pflanzen über einen längeren Zeitraum des aktiven Wachstums vor Insekten, welche die lebenden Pflanzen befallen, wobei das Verfahren dadurch gekennzeichnet ist, das man auf das Blattwerk der Pflanzen oder auf den Boden oder andere Medien in denen sie wachsen eine insektizid wirksame Menge einer Verbindung der folgenden Formel appliziert

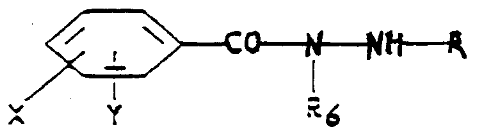

wobei R und $R_6$ jeweils unabhängig für Wasserstoff, $C_{2-6}$-Alkyl oder $C_{5-6}$-Cycloalkyl stehen; X und Y jeweils unabhängig für H, $C_{1-3}$-Alkyl, $C_{1-3}$-Alkoxy, $C_{1-3}$-Alkylthio, $C_{1-3}$-Alkylsulfinyl, $C_{1-3}$-Alkylsulfonyl, Cyano, F, Cl, Br, J, Nitro, $CF_3$, $R_1CF_2Z$-, 1,1-Difluor-2,2-dichlorethoxy, $R_2CO$ oder $R_3R_4N$ stehen und falls sie zusammengefaßt sind, X und Y einen Ring bilden können, in dem XY die folgende Struktur darstellt:

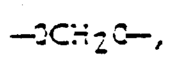 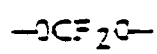 oder 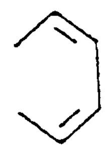 ;

Z für $S(O)_n$ oder O steht; $R_1$ für H, F, $CHF_2$, CHFCl oder $CF_3$ steht; $R_2$ für $C_{1-3}$-Alkyl, $C_{1-3}$-Alkoxy oder $R_3R_4N$ steht; $R_3$ für H oder $C_{1-3}$-Akyl steht; $R_4$ für H, $C_{1-3}$-Alkyl oder $R_5CO$ steht; $R_5$ für H oder $C_{1-3}$-Alkyl steht und n für 0, 1 oder 2 steht; mit den Maßgaben, daß dann, wenn R für H steht, $R_6$ für $C_{2-6}$-Alkyl oder $C_{5-6}$-Cycloalkyl steht, und daß dann, wenn $R_6$ für Wasserstoff steht, R für $C_{2-5}$-Alkyl oder $C_{5-6}$-Alkyl steht.

**5.** Verfahren gemäß Anspruch 4, wobei die Verbindung auf das Blattwerk der Pflanzen oder auf den Boden oder andere Medien in denen diese wachsen mit einer Rate von etwa 0,1 kg pro ha bis etwa 10,0 kg pro ha appliziert wird.

**6.** Verfahren gemäß Anspruch 4, wobei die Verbindung auf das Blattwerk der Pflanzen oder auf den Boden oder andere Medien in denen sie wachsen in der Form eines verdünnten Sprays, enthaltend etwa 10 ppm bis 10 000 ppm der Verbindung appliziert wird.

**7.** Verfahren, zum systemischen Schutz von Pflanzen vor Insektenbefall, wobei das Verfahren dadurch gekennzeichnet ist, daß man auf den Boden oder andere Medien, in denen die Pflanzen wachsen, eine systemisch wirksame insektizide Menge einer Verbindung der folgenden Formel appliziert

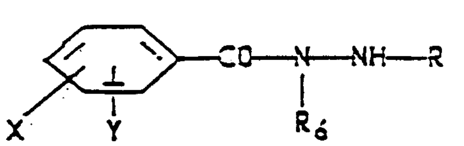 ,

wobei R und $R_6$ jeweils unabhängig für Wasserstoff, $C_{2-6}$-Alkyl oder $C_{5-6}$-Cycloalkyl stehen; X und Y jeweils unabhängig für H, $C_{1-3}$-Alkyl, $C_{1-3}$-Alkoxy, $C_{1-3}$-Alkylthio, $C_{1-3}$-Alkylsulfinyl, $C_{1-3}$-Alkylsulfonyl, Cyano, F, Cl, Br, J, Nitro, $CF_3$, $R_1CF_2Z$-, 1,1-Difluor-2,2-dichlorethoxy, $R_2CO$ oder $R_3R_4N$ stehen und falls sie zusammengefaßt sind, X und Y einen Ring bilden können, in dem XY die folgende Struktur darstellt:

$-OCH_2O-$, $-OCF_2O-$ oder 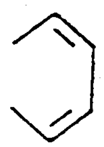 ;

Z für $S(O)_n$ oder O steht; $R_1$ für H, F, $CHF_2$, CHFCl oder $CF_3$ steht; $R_2$ für $C_{1-3}$-Alkyl, $C_{1-3}$-Alkoxy oder $R_3R_4N$ steht; $R_3$ für H oder $C_{1-3}$-Akyl steht; $R_4$ für H, $C_{1-3}$-Alkyl oder $R_5CO$ steht; $R_5$ für H oder $C_{1-3}$-Alkyl steht und n für 0, 1 oder 2 steht; mit den Maßgaben, daß dann, wenn R für H steht, $R_6$ für $C_{2-5}$-Alkyl oder $C_{5-6}$-Cycloalkyl steht, und daß dann, wenn $R_6$ für Wasserstoff steht, R für $C_{2-5}$-Alkyl oder $C_{5-6}$-Cycloalkyl steht.